# EUROPEAN PATENT SPECIFICATION

(11) **EP 0 403 832 B1**
(45) Date of publication and mention of the grant of the patent: **19.10.1994**
(21) Application number: 90110126.1
(22) Date of filing: 29.05.1990
(51) Int. Cl.: A61F 13/15

(54) **Disposable diaper**
Wegwerfwindel
Couche à jeter

(30) Priority: 29.05.1989 JP 62315/89
(43) Date of publication of application: 27.12.1990
(73) Proprietor: UNI-CHARM CORPORATION, Kawanoe-shi Ehime-ken (JP)
(72) Inventor: Suzuki, Migaku, Kanagawa-ken (JP); Nozaki, Satoshi, Umag-gun Ehime-ken (JP); Takeshi Kudo, Kawanoe-shi Ehime-ken (JP); Ohnishi, Kazuaki, Kagawa-ken (JP)
(74) Representative: Strehl Schübel-Hopf Groening & Partner

(56) References cited:
- EP-A- 0 109 126
- EP-A- 0 243 013
- EP-A- 0 329 160

## Description

The present invention is related to a disposable diaper in accordance with the introductory portion of Claim 1. A disposable diaper of this type is disclosed in EP-A-O 243 013.

The top sheet of said known diaper may be of fibrous non-woven fabric, porous plastic film etc. and the backsheet may be of plastic film or a laminate sheet consisting of saif plastic film and fibrous non-woven fabric. Each of said side flaps comprises a first side flap and a second side flap. Said first side flap extends laterally outwardly from said absorbent body and said second side flap extends from said first side flap in a laterally divergent manner and is made of an air-permeable and waterproof sheet, preferably of fibrous non-woven fabric which has been subjected to waterproofing with silicone resin. At least one of said elastic bands is associated with each of said second side flaps and acts thereon to cause at least an edge portion thereof to be held over at least a central portion of the length thereof in a substantially upstanding position relative to the first side flap from which it extends. These features are based on the object to achieve an effective seal around the thighs of a baby and thereby to minimize a possible leakage of excretions around the thighs.

### Background Of The Invention

For the conventional diapers of this type, the side wings protruding from the transverse side of the waist are usually formed as a part of the side flaps located at the two transverse sides of the diaper. Since the side flaps are made of a laminate consisting of a liquid-permeable top sheet and a liquid-barrier backsheet, the side flaps containing the side wings are not air permeable. This causes a warm dampness.

In recent years, an air-permeable plastic film has been used at said backsheet. However, since the front and back side wings are overlapped when the diaper is used, the air permeability is poor and warm dampness still exists. This disadvantage is particularly significant in the summer. Since a lot of sweat is generated on the skin of the wearer in the summer, the wearer feels uneasy when said topsheet is hydrophobic.

The purpose of this design is to overcome the disadvantage of the conventional scheme by providing a type of disposable diaper and the top layer of the side wings having sweat-absorptive and air permeable ability and with the back layer of the side wings having liquid-barrier and air-permeable ability.

### Summary Of The Invention

In order to realize the aforementioned purpose, this invention provides an improvement of the above-mentioned known disposable diaper as disclosed in the characterizing portion of claim 1.

Thus, the invention provides a type of disposable diaper consisting of a liquid-permeable topsheet in contact with the skin (of the wearer), a liquid-barrier backsheet not in contact with the skin, a liquid-absorptive core sandwiched between these two sheets, side flaps extending from the two side edges of the core and having elastic parts stretchable in the longitudinal direction, and side wings protruding from the two sides of the front and rear waist portions.

Said side wings are made of a top layer in contact with the skin and a back layer not in contact with the skin. Said top layer can absorb sweat and is air permeable. Said back layer is a liquid barrier but is air permeable.

In a preferable application status, said side flaps are made of the same materials as said side wings, and the side wings are formed as a part of the side flaps.

The other preferable application status will be explained in the following with reference to application examples.

### Brief Description Of The Drawings

FIGURE 1 is a developed plan view of the diaper of this design.

FIGURE 2 is a cross-sectional view cut along line II-II in FIGURE 1.

FIGURE 3 is a plan view of the example for continuous formation of a portion of the side flaps.

FIGURE 4 is a cross-sectional view cut along line IV-IV in Figure 3.

FIGURE 5 is a developed plan view of the composite web cut at the length of a single diaper formed in the formation process shown in Figure 3.

FIGURE 6 is a cross-sectional view cut along line VI-VI in FIGURE 5.

### Application Examples

In the following, the application examples of the diaper of this design will be explained with reference to figures.

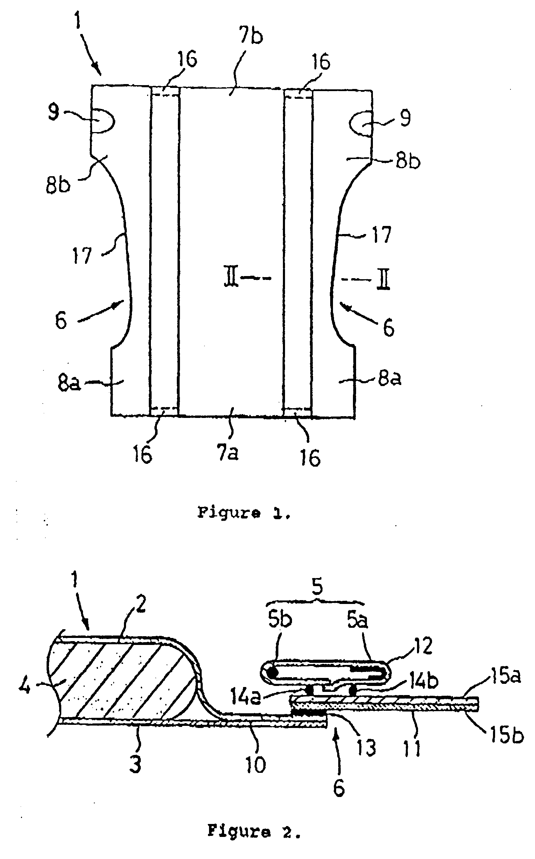

As shown in Figures 1 and 2, diaper (1) is made of liquid-permeable topsheet (2) in contact with the skin, liquid-barrier backsheet (3) not in contact with the skin, liquid-absorptive core (4) sandwiched between two sheets (2), (3), side flaps (6) which are extended from the two side edges of the core and having elastic part (5) stretchable in the longitudinal direction, and side wings (8a), (8b) protruding from the two side edges of front and rear waist portion (7a), (7b). On side wing (8b) of rear waist portion (7b), pressing tape fastener (9) is mounted to fix on backsheet (3) of front waist (7a) when diaper (1) is assembled.

Each side flap (6) contains a first portion (10) made of top sheet (2) and backsheet (3) extending from the side edge of core (4) and bonded with each other, a second portion (11) which is bonded with the outer edge of the first portion via adhesive (13), and a third portion (12) which is bonded with the second portion via adhesives (14a), (14b) near the inner edge of the second portion.

Second portion (11) is made of a composite sheet consisting of top layer (15a) in contact with the skin and back layer (15b) not in contact with the skin. Top layer (15a) can absorb sweat and is air permeable. On the other hand, back layer (15b) is liquid-barrier but air-permeable. Top layer (15a) is made of sweat-absorptive nonwoven fabrics, such as spun-bond, melt-bond, melt-blown, or other nonwoven fabrics of polyester fibers with the fiber surfaces processed to become hydrophilic; nonwoven fabrics of polyester, polypropylene, and other hydrophobic fibers interwoven with said hydrophilic processed fibers, rayon, and other cellulose-series hydrophilic fibers using the water jet scheme; as well as the melt-bond nonwoven fabrics made of a mixture of the hydrophilic processed fibers or hydrophilic fibers and said hydrophobic fibers. It is preferred that the amount of the hydrophilic processed fibers of the hydrophic fibers with respect to the hydrophobic fibers be larger than 70%. Back layer (15b) is made of the spun-bond, melt-bond, or melt-blown nonwoven fabrics of polyester, polypropylene, etc., as well as polyethylene or other porous plastic film. Here, the porosity refers to the air permeability and [liquid] barrier properties acquired by slitting or punching the film. Top layer (15a) and back layer (15b) can be bonded using heating welding, ultrasonic welding or an adhesive (hot-melt type).

Third portion (12) is formed in a folded sleeve shape and is bonded with second portion (11). In addition, at two ends (16) in the longitudinal direction, it is bonded with first portion 10, and second portion (11). At the two side edges within the sleeve, elastic parts (5a), (5b) are attached while they are being stretched in the longitudinal direction. Elastic parts (5a), (5b) then contract, as a result, the two side edges of third portion (12) rise with respect to bonding portions (14a), (14b); the entire third portion (12) is deformed into a cross-sectional shape of U or V, with its two side edges pressing on the thighs of the wearer to prevent leakage of the body fluid. Preferably, third portion (12) is formed from the nonwoven fabrics of polyester, polypropylene, or other hydrophobic fibers. However, for this design, third portion (12) is not indispensable. It is also possible not to set a third portion (12), instead, an elastic part can be arranged along recession portion (17) of second portion (11) for press-sealing the thigh portion.

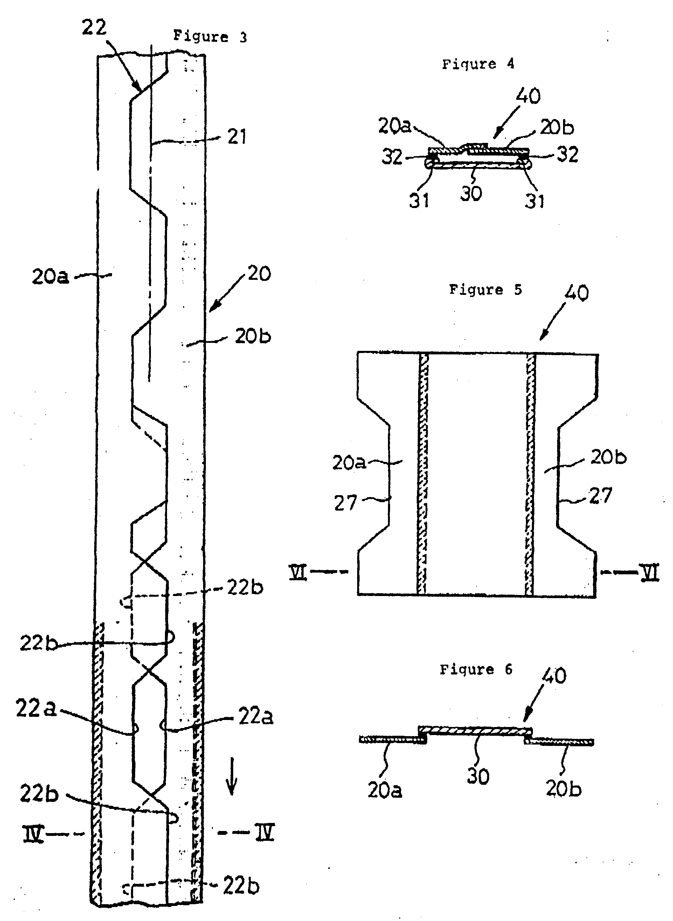

Recessed portion (17) arranged on side flap (6) can improve the fit of the diaper on the wearer's body. This recessed portion (17) can be realized by cutting off a portion of the sheet forming side flap (6). However, in order to reduce the cost of the diaper by reducing the amount of the starting material sheet, as shown in Figures 3-6, it is preferred that second portion (11) be formed as an integral part of side flap (6) and made to be bonded with topsheet (2) and/or backsheet (3).

One example of this scheme can be explained as follows. A continuous first web (20) is provided which comprises a laminate or composite of a sweat-absorptive and air-permeable layer, and a liquid barrier but air-permeable layer, such as described above for second portion (11). As shown in Figure 3, continuous first web (20), which is to form second portion (11), has substantially parallel side edges, cutting lines (22) forming concave-convex portions via longitudinal central line (21) are cut by a roll cutter (not shown in the figure) in a periodically repeated manner in the longitudinal direction. In this way, first and second divided webs (20a), (20b) having concave edge portion (22a) and convex edge portion (22b) are formed, and at the same time, recessed portion 27 is formed. First division web (20a) is separated from second division web (20b), and it is deviated in the longitudinal direction by a pitch of (1/2)n (here, n is an odd number) with respect to second division web (20b). Concave edge portions (22a) and convex edge portions (22b) of first and second division webs (20a), (20b) are opposited and matched to each other.

On the other hand, for second web (30) to be used to form topsheet (2) or backsheet (3), its two sides are folded back to the upper surface; adhesive (32) is coated on fold-back portion (31) and it is positioned on the lower surface of first and second division webs (20a), (20b) matched with each other as pointed out above. As shown in Figure 4, the outer edges of first and second division webs (20a), (20b) and the fold-back portions (31) of second web (30) are bonded with adhesive (32). In this way, continuous composite web (40), is formed from first and second division webs (20a), (20b) and second web (30). As shown in Figures 5 and 6, for composite web 40, first and second division webs (20a), (20b) are developed outwards and combined with the other structural parts of the diaper, followed by cutting at the prescribed portion.

### Effects Of The Design

For the diaper of this design, the top layer of the side wings protruding from the two side edges of the side flaps in contact with the skin can absorb sweat and are air permeable; on the other hand, the back layer not in contact with the skin is a liquid barrier but is air permeable. When the diaper is worn, the overlapped front and rear side wings is contact with the skin are still air permeable. In this way, the warm dampness can be significantly suppressed, the sweat can be absorbed, and the body fluid reaching the side wing region cannot leak out.

## Claims

1. Disposable diaper comprising:
a liquid-permeable topsheet (2) for contact with the skin of the wearer;
a liquid barrier backsheet (3);
a liquid absorptive core (4) sandwiched between the topsheet (2) and the backsheet (3);
a pair of elasticized side flaps (6) extending longitudinally of said diaper at respective opposite side edges thereof;
each of the side flaps (6) including portions of said topsheet (2) and said backsheet (3) which extend from the respective side edge of said absorbent core (4), and elastic means (5) extending generally at respective side edges of said topsheet (2) and said backsheet (3) of said side flaps (6) for elastically contracting said side flaps (6) the elastic means being stretchable in the longitudinal direction,
each of said side flaps (6) comprising a side wing (8a, 8b) extending laterally from the respective elastic means (5);
each of said side wings (8a, 8b) extending laterally of at least one of the front and the rear waist portions (7a, 7b) of said diaper,
each of said side wings (8a, 8b) comprising a composite two-layer sheet (11), including an air-permeable, sweat-absorptive top layer (15a) for contact with the skin of the wearer, and an air-permeable, liquid barrier back layer (15b), not in contact with the skin;
characterized in that
said side wings (8a, 8b) extend laterally from the side edges of said top sheet (2) and said back-sheet (3) of said side flap (6),
each of said side wings (8a, 8b) is joined with adhesives (13) to said side edges of said top sheet (2),
each of said elastic means (5) comprises an outer covering sleeve (12), and includes a pair of elastic elements (5a, 5b) extending within said outer covering sleeve (12),
said outer covering sleeve (12) of each said elastic means (5) is bonded with adhesives (14a, 14b) at the top layer (15a),
each of said elastic means (5) extending generally along opposite sides of the respective adhesives (14a, 14b) for said covering sleeve (12), so that contraction of said elastic elements (5a, 5b) results in said covering sleeve (12) having the generally U-shaped cross-sectional shape.

2. Disposable diaper according to claim 1, characterized in that said top layer (15a) of each of said side wings (8a, 8b) is made of nonwoven fabric containing more than 70% hydrophilic fibers.

3. Disposable diaper according to claim 1, wherein said back layer (15b) of each of said side wings (8a, 8b) comprises hydrophobic nonwoven fabric.

## Patentansprüche

1. Wegwerfwindel mit:
einer flüssigkeitsdurchlässigen oberen Schicht (2) zum Kontakt mit der Haut des Benutzers;
einer flüssigkeitssperrenden hinteren Schicht (3);
einem flüssigkeitsabsorbierenden Kern (4), der zwischen der oberen Schicht (2) und der hinteren Schicht (3) angeordnet ist;
einem Paar elastisch ausgebildeter Seitenlaschen (6), die sich in Längsrichtung der Windel entlang ihrer gegenüberliegenden Seitenränder erstrecken;
wobei jede Seitenlasche (6) Teile der oberen Schicht (2) und der hinteren Schicht (3), die von dem jeweiligen Seitenrand des absorbierenden Kerns (4) abstehen, sowie elastische Mittel (5) umfaßt, die etwa an den jeweiligen Seitenrändern der oberen Schicht (2) und der hinteren Schicht (3) der Seitenlaschen (6) vorgesehen sind, um die Seitenlaschen (6) elastisch zusammenzuziehen, wobei die elastischen Mittel (5) in Längsrichtung dehnbar sind,
jede Seitenlasche (6) einen Seitenflügel (8a, 8b) aufweist, der sich seitlich der entsprechenden elastischen Mittel (5) erstreckt;
wobei sich jeder Seitenflügel (8a, 8b) seitlich eines vorderen und/oder hinteren Taillenabschnittes erstreckt,
und jeder Seitenflügel (8a, 8b) eine zweilagige Verbundschicht (11) aufweist, die eine luftdurchlässige, schweißabsorbierende obere Schicht (15a) zum Kontakt mit der Haut des Benutzers, sowie eine luftdurchlässige, flüssigkeitssperrende, nicht mit der Haut des Benutzers in Kontakt tretende, hintere Schicht (15b) einschließt,
dadurch gekennzeichnet, daß
die Seitenflügel (8a, 8b) seitlich von den Seitenrändern der oberen Schicht (2) und der hinteren Schicht (3) der Seitenlasche (6) abstehen,
die Seitenflügel (8a, 8b) mittels Klebstoff (13) an den Seitenrändern der oberen Schicht (2) angebracht sind, jedes der elastischen Mittel (5) eine äußere Umhüllung (12) umfaßt und ein Paar elastischer Glieder (5a, 5b) einschließt, das sich innerhalb der äußeren Umhüllung (12) befindet,
wobei die äußeren Umhüllung (12) mittels Klebstoff (14a, 14b) an der oberen Schicht (15a) befestigt ist, und sich jedes der elastischen Mittel (5) etwa entlang gegenüberliegender Seiten der jeweiligen Kleber (14a, 14b) für die Umhüllung (12) erstreckt, so daß ein Zusammenziehen der elastischen Glieder (5a, 5b) dazu führt, daß die Umhüllung (12) eine etwa U-förmige Querschnittsform annimmt.

2. Wegwerfwindel nach Anspruch 1, dadurch gekennzeichnet, daß die obere Schicht (15a) jedes Seitenflügels (8a, 8b) aus einem Vlies besteht, das mehr als 70% hydrophile Fasern enthält.

3. Wegwerfwindel nach Anspruch 1, dadurch gekennzeichnet, daß die hintere Schicht (15b) jedes Seitenflügels (8a, 8b) ein hydrophobes Nonwoven-Vlies enthält.

## Revendications

1. Couche jetable comportant :
une feuille supérieure (2) perméable au liquide destinée à être en contact avec la peau du porteur,
une feuille arrière (3) formant barrière au liquide,
un noyau (4) absorbant un liquide, enserré entre la feuille supérieure (2) et la feuille arrière (3),
une paire de volets latéraux (6) munis d'élastiques s'étendant longitudinalement par rapport à ladite couche au niveau de bords latéraux respectifs opposés de celui-ci,
chacun des volets latéraux (6) comportant des parties de ladite feuille supérieure (2) et de ladite feuille arrière (3) qui s'étendent à partir du bord latéral respectif dudit noyau absorbant (4), et des moyens élastiques (5) s'étendant de manière générale au niveau des bords latéraux respectifs de ladite feuille supérieure (2) et de ladite feuille arrière (3) desdits volets latéraux (6) pour contracter de manière élastique lesdits volets latéraux (6), les moyens élastiques pouvant être étirés dans la direction longitudinale, chacun desdits volets latéraux (6) étant constitué d'une aile latérale (8a, 8b) s'étendant latéralement à partir des moyens élastiques respectifs (5),
chacune desdites ailes latérales (8a, 8b) s'étendent latéralement à partir d'au moins une des parties avant et arrière (7a, 7b) formant taille de ladite couche,
chacune desdites ailes latérales (8a, 8b) étant constituée d'une feuille (11) composite à deux couches, comportant une couche supérieure (15a) perméable à l'air absorbant la transpiration, destinée à être en contact avec la peau du porteur et une couche arrière (15b) perméable à l'air, formant barrière au liquide, qui n'est pas en contact avec la peau,
caractérisée en ce que
lesdites ailes latérales (8a, 8b) s'étendent latéralement à partir des bords latéraux de ladite feuille supérieure (2) et de ladite feuille arrière (3) dudit volet latéral (6),
chacune desdites ailes latérales (8a, 8b) est reliée à l'aide d'adhésif (13) auxdits bords latéraux de ladite feuille supérieure (2),
chacun desdits moyens élastiques (5) comporte un manchon (12) de recouvrement extérieur, et comporte une paire d'éléments (5a, 5b) formant élastiques s'étendent à l'intérieur dudit manchon (12) de recouvrement extérieur,
ledit manchon (12) de recouvrement extérieur de chacun desdits moyens élastiques (5) est fixé à l'aide d'adhésifs (14a, 14b) au niveau de la couche supérieure (15a)
chacun desdits moyens élastiques (5) s'étendent de manière générale le long de côtés opposés des adhésifs respectifs (14a, 14b) dudit manchon de recouvrement (12), de sorte qu'une contraction desdits éléments (5a, 5b) formant élastiques entraîne que ledit manchon de recouvrement (12) ait de manière générale une forme de U en coupe transversale.

2. Couche jetable selon la revendication 1, caractérisée en ce que ladite couche supérieure (15a) de chacune desdites ailes latérales (8a, 8b) est constituée d'un tissu non-tissé contenant plus de 70% de fibres hydrophiles.

3. Couche jetable selon la revendication 1, dans laquelle ladite couche arrière (15b) de chacune desdites ailes latérales (8a, 8b) est constituée d'un tissu non-tissé hydrophobe.
